# EUROPEAN PATENT APPLICATION

(11) **EP 0 537 827 A1**
(43) Date of publication of application: **21.04.1993**
(21) Application number: 92203043.2
(22) Date of filing: 03.10.1992
(51) Int. Cl.: G01N 33/543, G01N 33/58, G01N 33/569

(54) **Method, test device and kit for assay of specific binding ligand using controlled flow through filtration membrane**

(30) Priority: 08.10.1991 US 773394
(71) Applicant: EASTMAN KODAK COMPANY, Rochester, New York 14650-2201 (US)
(72) Inventor: Contestable, Paul Bernard, c/o Eastman Kodak Co., Rochester, New York 14650-2201 (US); Snyder, Brian Anthony, c/o Eastman Kodak Co., Rochester, New York 14650-2201 (US); Pelanek, Geraldine Ann, c/o Eastman Kodak Co., Rochester, New York 14650-2201 (US); Sutton, Richard Calvin, c/o Eastman Kodak Co., Rochester, New York 14650-2201 (US)
(74) Representative: Nunney, Ronald Frederick Adolphe

(57) **Abstract**

A test device has a microporous filtration membrane and absorbing means in fluid contact therewith which are designed so as to provide a drainage time of at least about 20 seconds for a given fluid sample. Retaining the fluid on the membrane for a longer period of time during a specific binding assay reduces background and improves assay sensitivity. The test device can be supplied as part of a diagnostic test kit and used in a variety of diagnostic methods. In particular, the invention is useful for the determination of microorganisms associated with periodontal diseases.

## Description

The present invention relates to a diagnostic method for the determination of a specific binding ligand in a specimen. It also relates to a test device and test kit which can be used in carrying out the method of this invention. This invention is particularly useful in the determination of microorganisms associated with periodontal diseases.

There is a continuous need in medical practice, research and diagnostic procedures for rapid, accurate and qualitative or quantitative determinations of biological substances which are present in biological fluids at low concentrations. For example, the presence of drugs, narcotics, hormones, steroids, polypeptides, prostaglandins or infectious organisms in blood, urine, saliva, vaginal secretions, dental plaque, gingival crevicular fluid and other biological specimens has to be determined in an accurate and rapid fashion for suitable diagnosis or treatment.

To provide such determinations, various methods have been devised for isolating and identifying biological substances employing specific binding reactions between the substance to be detected (sometimes identified as a "ligand") and a compound specifically reactive with that substance (sometimes identified as a "receptor").

Specific microorganisms have been implicated as indicators for a number of periodontal diseases in humans and animals, such as gingivitis and periodontitis. The importance of such diseases is growing in the human population, especially as people live longer, and prevention of such diseases is becoming of considerable importance to dentists, insurance carriers and the health industry in general. In addition, proper dental care for animals is a growing concern in our culture.

Detection of microorganisms associated with periodontal diseases has been accomplished using culture techniques, DNA probes and a number of immunological procedures, such as agglutination assays, enzyme linked immunosorbent assays (ELISA) and others known in the art. ELISA assays utilize the reaction of an extracted antigen from the microorganism(s) and the corresponding antibody to form an immunological complex. As noted above, usually uncomplexed materials are washed from the complex in order to provide an accurate assay result.

An advance in the art in the detection of microorganisms associated with periodontal diseases is described and claimed in EP-A-0 439 210. This case describes the simultaneous detection and differentiation of these microorganisms, and particularly Actinobacillus actinomycetemcomitans, Porphyromonas gingivalis and Prevotella intermedia, in an immunometric (also known as "sandwich") assay using water-insoluble reagents in defined regions of a microporous filtration membrane. The simultaneous detection and differentiation of these microorganisms have considerable clinical and commercial significance.

The assay described in EP-A-0 439 210 is able to detect a plurality of microorganisms simultaneously. For example, three different serotypes from each of the three microorganisms noted above can be detected simultaneously. In order to do that, however, it was found desirable to use a larger test well in the disposable test device (similar to that commercially available under the SURECELL trademark from Eastman Kodak Company). When this was done, however, the flow of fluid during the washing of uncomplexed materials through the membrane was less controllable. In addition, the presence of a large number of different antibodies in close proximity on the same membrane gave rise to an increase in non-specific binding. All of these problems, while small individually, combined to increase the background signal undesirably. The result was a decrease in assay sensitivity due to weak specific signals (at low antigen concentration) being hidden by high non-specific background signal on the membrane. Another evidence of a problem with the former assay is the observed difficulty in visualizing true positive signals due to high background levels.

Thus, there was a desire to solve the noted high background problem when maintaining the advantages presented by the multiple testing of antigens using the invention described in EP-A-0 439 210.

US-A-4,923,680 describes the use of a polymer coating on membranes in test devices to provide an "induction time" (time for fluid flow through the membrane) to be from about 30 to about 300 seconds for a given liquid pressure. There is no mention that this would solve a background problem or reduce non-specific binding. Moreover, it would be desirable to increase the flow time in certain instances without having the additional requirements of coating the polymer on the membrane during manufacturing. The presence of a polymer on the membrane may further contribute to non-specific binding in certain instances and creates additional manufacturing problems, and these are to be avoided as well.

The problems of high background in assays for specific binding ligands of interest have been overcome with a method for the determination of a specific binding ligand comprising the steps of:
A. forming an insolubilized complex of a target specific binding ligand and a receptor specific therefor on one surface of a microporous filtration membrane which is in fluid contact, on the opposing surface thereof, with a means for absorbing fluid passing through the membrane,
B. removing uncomplexed materials from the insolubilized complex by washing them through the membrane into the absorbing means with a wash solution comprising a wash reagent,
   provided that the average pore size of the membrane and the density or hydrophilicity of the absorbing means are such that, the time for washing is at least 20 seconds, and
C. detecting the insolubilized complex on the membrane as an indication of the determination of the target specific binding ligand.

This invention also provides a test device comprising a test well which comprises:
(a) an upper reaction compartment,
(b) a lower fluid collection compartment, and
(c) a microporous filtration membrane separating the compartments,
   the lower fluid collection compartment containing means for absorbing fluid from the upper compartment to the lower compartment through the membrane, the absorbing means being in fluid contact with the membrane,
   the test device characterized wherein the membrane has an average pore size and the absorbing means has a density or hydrophilicity such that, the time for a given volume of fluid passing through the membrane is at least 20 seconds.

Further, a diagnostic test kit comprises:
(1) receptor molecules for a target specific binding ligand, and
(2) a wash solution comprising a wash reagent,
   the test kit characterized wherein it further comprises a test device as described above.

The present invention provides an improved method for the sensitive detection of various specific binding ligands, such as microorganisms associated with periodontal diseases. The assay can be used to advantage for rapid and sensitive detection of the ligand using a microporous filtration membrane to collect the specific binding complex while uncomplexed materials are drained therethrough. The background noted with earlier assays is greatly reduced, increasing sensitivity to low concentrations of target ligand. This allows for the detection of multiple ligands in the same test well with greater confidence in the results.

These advantages have been achieved with a decrease in the flow rate of the wash solution to remove uncomplexed materials. One would expect that better sensitivity and reduced background could be achieved by increasing the wash volume or by increasing the rate of flow. Unexpectedly, this is not the case.

We have found that the flow rate can be critically controlled by either adjusting the pore size of the membrane, or the density or hydrophilicity of the absorbing means which is in fluid contact with the membrane, or adjusting both, such that, for a given volume of fluid, the washing time is at least about 20 seconds. The details of these adjustments are described below. The adjustment of the pore size is more important, but in instances where that is not possible or not completely effective, the absorbing means can also be adjusted to achieve the desired slow flow rate and improved assay results. Moreover the advantages of this invention are achieved without the use of polymer coatings on the membrane, contrary to the teaching of US-A-4,923,680.

FIG. 1 is a vertical section view, partially schematic, of a test device comprising a single test well according to this invention.

FIG. 2 is a perspective illustration of a preferred test device according to this invention.

FIG. 3 is a graphical representation of the background signals for various test devices used in assays as described in Examples 1-5 below.

FIG. 4 is graphical representation of the flow rate of fluid samples through the various test devices used in assays as described in Examples 1-5 below.

FIG. 5 is a graphical plot of background density values versus fluid flow rate for various test devices used in assays as described in Examples 1-5 below.

The present invention provides a test device and diagnostic test kit that can be used in any specific binding assay whereby a targeted specific binding ligand is to be detected. Specific binding ligand is normally defined in the art as any chemical or biological material for which there exists or can be prepared a receptor molecule which will complex specifically with that ligand. Many such specific binding pairs are known, including antibody-antigen, antibody-antibody, sugar-lectin, avidin-biotin and others readily apparent to one skilled in the art. In the context of this invention, a nucleic acid is also considered a specific binding ligand which "complexes" (that is, hybridizes) with a complementary nucleic acid which is considered the receptor molecule.

In most cases, however, the ligand is extracted from a microorganism (or other organism or component thereof), virus particle, protozoan, or other cellular material, complexed with its corresponding receptor, and the complex is detected in a suitable manner.

The method of this invention can be used to assay any human or animal biological fluid or specimen of interest including, but not limited to, whole blood, plasma, sera, lymphatic fluid, bile, urine, spinal fluid, seminal fluid, vaginal secretions, sputum, perspiration, stool specimens, fluid preparations of tissues, periodontal tissue, dental plaque, crevicular fluid and saliva.

Once the ligand is available for complexation, it is reacted with its corresponding receptor. The complex can be formed in solution and then added to the test device for "capture" on the membrane.

Preferably, the complex is formed on the membrane in some fashion by bringing the specific binding ligand and receptor into contact on the membrane. Contact can occur at a wide range of temperatures, for example from below room temperature to those near the boiling point of water as long as the components of the complex are not advsersely affected. Generally, the contacting is carried out in a range of from 15 to 30°C with room temperature more likely.

Various assay protocols can be used in the practice of the invention including those known in the art as competitive immunoassays and enzyme-linked immunosorbent assays.

In one preferred embodiment, the target specific binding ligand (for example an extracted antigen or nucleic acid) can be insolubilized by direct adsorption or covalent attachment to the microporous filtration membrane. Such assays are generally known in the art as "direct binding" assays whereby the specific binding ligand directly binds to the material, and labeled receptor molecules (such as antibodies or complementary nucleic acid) are used to complex with the insolubilized ligand. Alternatively, the receptor can be unlabeled and a second receptor which is labeled and specific to the first receptor can then be used.

A most preferred embodiment of this invention is an immunometric or sandwich assay in which the specific binding ligand is reacted at different epitopic sites with two receptor molecules, one of which is suitably labeled, and the second being immobilized on a water-insoluble substrate (or capable of being immobilized such as through avidin-biotin complexation).

Preferably, the ligand is immobilized on particulate materials as described above. These particles are more preferably polymeric, spherical in shape and have an average particle size of from 0.01 to 10 µmeters. The resulting reagent of receptor attached to particles can be added to the test device prior to, simultaneously with or subsequently to addition of the specific binding ligand.

The insolubilizable receptors used in the immunometric assays can be attached to water-insoluble materials by physical or chemical means, including adsorption or covalent reaction with reactive groups on the surface of the materials. Covalent attachment is preferred for better assay sensitivity.

Particularly useful particulate materials are polymeric beads which are described, for example, in EP-A-0 323 692 and which are prepared from one or more ethylenically unsaturated polymerizable monomers having an active halo atom, activated 2-substituted ethylsulfonyl or vinylsulfonyl groups. Other particularly useful particles having reactive carboxy groups are described in copending EP-A-0 466,220.

The receptor can also be designed for being insolubilized using a specific binding complex such as avidin-biotin, a sugar with a lectin or others known in the art. Avidin-biotin reactions are preferred in these embodiments because of the high affinity the components have for each other. The receptor molecule to be insolubilized can be biotinylated (such as a biotinylated antibody or nucleic acid ), and before, during or after its reaction with the ligand, it can be complexed with avidin which is attached to the membrane or other solid material in a test well. The fluid in the specimen can dislodge the receptor for complexation with the ligand.

Most preferably, the insolubilized receptors described above are coated or deposited on the microporous filtration membrane, Useful membrane materials include, but are not limited to, porous natural or synthetic polymers, sintered glass, membranes of glass or polymeric films or fibers, ceramic materials, cellulosic materials and particulate structures composed of beads bound together with an adhesive or binder material.

If desired, the membrane can be coated with surfactant or nonimmunoreactive protein (such as casein or succinylated casein). However, such coatings are not designed to slow down filtration time like the polymer coatings described in US-A-4,923,680. Preferably, however, the membrane is uncoated with surfactant, protein or polymer of any type (except that used in admixture with immobilized receptor).

The immobilized receptor can be affixed to the membrane over its entire surface or in defined regions thereof.

It is critical to this invention that washing time be at least 20 seconds for a given fluid volume at room temperature. Thus, the time of drainage through the membrane for the wash fluid is at least 20 seconds. The wash time should be at least 20 seconds no matter what the membrane area and wash volume are. Thus, one skilled in the art could readily determine an appropriate membrane pore size, absorbant density for a given fluid volume and membrane area to achieve the desired "slow" drainage, depending upon such factors as the type of assay, the design of the test device and the particular target ligand and its binding capacity to the receptor molecules. In most instances, the amount of fluid will vary from 30 to 1000 µl with a sample of from 400 to 600 µl being most preferred, especially when using the preferred test devices described below. Generally, the membrane area contacted with the wash fluid is in the range of from 25 to 250 mm² with an area of from 50 to 125 mm² being preferred.

To achieve this desired washing time, the average pore size of the membrane in the largest dimension and the density or hydrophilicity of the absorbing means (described below) are specifically chosen for the desired result. In preferred embodiments, the pore size of the membrane is used as the controlling parameter. The density or hydrophilicity of the absorbing means can be a desirable parameter for washing time control as well. There are many combinations of membrane pore size and absorbing means that can be devised to achieve this result, and one skilled in the art could carry out routine experimentation for that purpose. While representative ranges of each parameter are taught herein, it should be understood that acceptable results can be achieved using one or both parameters outside the indicated ranges.

Generally, the average pore size of the membrane is from 0.1 to 3 µmeters. A preferred range is from 0.8 to 1.4 µmeters with 1.2 µmeters being most preferred.

The insolubilized complex is formed on one surface of the membrane, and an absorbing means is in fluid contact with the opposing surface of the membrane. The absorbing means is generally any suitable bibulous material which has sufficient porous volume to soak up the specimen sample and all fluids used during the assay. Useful absorbing means include, but are not limited to, conventional fibrous and sponge-like materials (such as cellulose acetate, glass, rayon and cotton fibrous wadding), elastomeric sponges, porous polymers and others readily apparent to one skilled in the art. A preferred material is cellulose acetate and can be obtained commercially having various densities from American Filtrona Company. The absorbing means generally has a density of from 1.2 to 2.5 g/cm³. However, absorbing means having densities outside this range can be used if the hydrophilicity of the material is such that it acts as if the density was inside the range. In other words, some less dense or more dense absorbing means may have a low hydrophilicity such that fluid is drawn slowly through the membrane to achieve the 20 seconds minimum drain time.

The absorbing means is in "fluid contact" with a side of the membrane. This means that fluid can readily flow through the membrane into the absorbing means. This may be accomplished by using a structural feature which channels the fluid through the membrane into the absorbing means. Preferably, the absorbing means is in physical contact with the membrane and fluid flows into it through the membrane at least by capillary action. Gravity may also be a principal factor in the movement of fluid into the absorbing means. Vacuum may be used in other embodiments to facilitate fluid flow.

Once complexation has occurred, uncomplexed materials and extraneous material from the specimen are washed through the membrane using a suitable washing solution.

As noted above, the time for the wash fluid to pass through the membrane is controlled so that it is at least about 20 seconds. Preferably, however, the time is within the range of from 25 to 45 seconds, with 35 seconds being optimum for a wash solution of 500 µl. For other volumes of fluids, the preferred and optimum flow times may be different.

The insolubilized complex is then detected in a suitable manner on the membrane. Preferably, the complex is detected by reacting the ligand with a labeled receptor. Such labels can include, but are not limited to, enzymes, radioisotopes, fluorogens, chromogens, biotin, avidin and other detection signal producing moieties that would be apparent from the voluminous literature about this technology. Detection systems for each type of label are well known.

More preferably, the signal produced from the complex is a colorimetric, fluorometric or chemiluminescent signal provided from an enzyme label and its reaction with one or more reagents, including a substrate therefor.

In the preferred immunometric assay of this invention, the ligand is complexed with two receptors, one insolubilized (as described above), and the other being detectably labeled. The order of contact of the receptors with the ligand is not critical, although it is preferred that the ligand be insolubilized prior to complexation with the labeled receptor. In the preferred embodiment of this immunometric assay, the ligand is an antigen and the two receptors are antibodies specific thereto and reactive at different epitopic sites on the antigen.

More preferably, the assay is used to detect antigen extracted from microorganisms associated with periodontal diseases. In particular, the microorganisms Actinobacillus actinomycetemcomitans, Porphyromonas gingivalis (formerly known as Bacteroides gingivalis) and Prevotella intermedia (formerly known as Bacteroides intermedius) are determined, either individually or collectively. However, other microorganisms which are suspected of being associated with periodontal diseases can also be detected or differentiated with this invention. Such other microorganisms include, but are not limited to, Wolinella recta, Bacteroides forsythus, Eikenella corrodens, Fusobacterium nucleatum and Troponema denticola.

The present invention can be carried out using a hand held membrane which is in fluid contact with the absorbing means, if desired. However, preferably, the membrane is disposed or mounted in a water-soluble test device or article having a suitable frame, compartments and structure for holding the membrane and fluid which is drained therethrough. Many such test devices are known in the art.

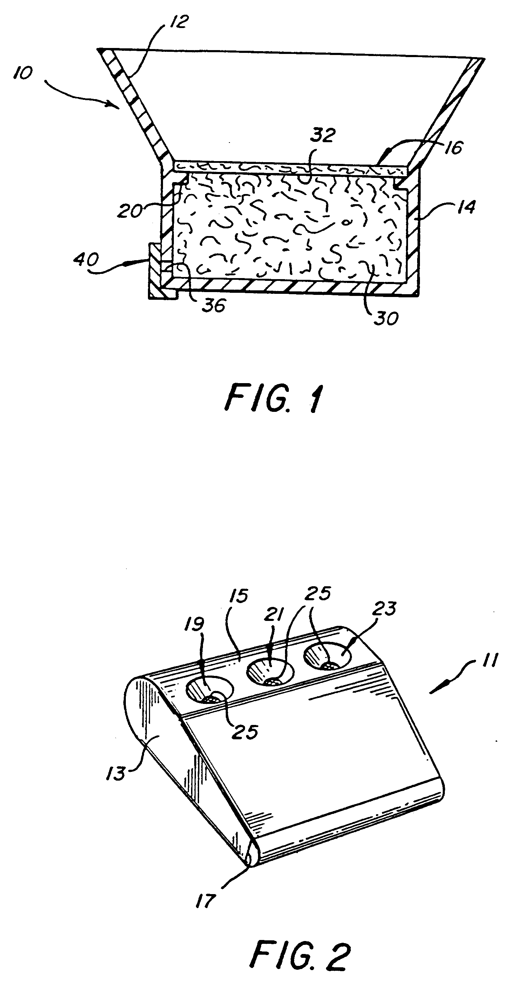

A representative test device containing a single test well is illustrated in FIG. 1. Test well **10** prepared in accordance with this invention has an upper reaction compartment **12**, a lower fluid collection compartment **14**, and a microporous filtration membrane **16** disposed between and separating the two compartments. Suitable means, such as a ledge **20**, can be used to hold membrane **16** in place. Membrane **16** is shown uncoated as described above.

Absorbing means **30** is provided in compartment **14** in fluid (for example, physical) contact with the bottom surface **32** of membrane **16**. Useful absorbing means are described above. To vent compartment **14** to the atmosphere, aperture **36** is provided. A suitable cover, such as hinged cover **40**, is provided over aperture **36**. There are various alternatives to hinged cover **40**, including a sliding cover (not shown). If only a single incubation of fluid on membrane **16** is required in the assay, hinged cover **40** can be omitted since fluid flow will be delayed from the designed combination of the pore size of membrane **16** and the density of absorbing means **30**.

It is advantageous to have two or more of the test wells shown in FIG. 1 joined together in a single test device. For example, FIG. 2 illustrates a disposable test device having three test wells designed for providing both negative and positive control results as well as a specimen test result.

More particularly in FIG. 2, test device **11** has frame **13** having a top surface **15** and front edge **17**. Test wells **19**, **21** and **23** each contain a microporous filtration membrane **25** (partially obscured) like that shown in FIG. 1 above through which fluids and uncomplexed materials flow during an assay into lower fluid collecting compartments (not shown).

As noted above, the test device can have test wells designed for both positive and negative controls. The purpose of a positive control is to produce a detectable signal (for example, a color change) that will always occur if the assay is carried out properly. Thus, the positive control indicates that the device and reagents are functioning so that, if the target ligand is present in the test specimen, there will also be a similar detectable signal produced. The purpose of the negative control is to detect any error or interferent in the assay that produces a signal even in the absence of the target ligand.

Detectable signals in the positive control test well can be the same or different from that expected from the test specimen. For example, the target ligand may produce a detectable dye while the positive control may cause a loss in dye signal. Obviously, for practical reasons, the signals are designed to be the same, and the types of reagents needed are described above in general and specific details are well known in the art. The positive control signal can be produced uniformly over the surface of the membrane in the test well, or it can be produced in predetermined patterns.

In another embodiment especially where multiple ligands are detected simultaneously, the positive control well has a multiplicity of regions with the same or different signals provided to indicate that the assay was carried out properly for each ligand assayed.

In general, the positive control test well has therein both target ligand and receptor therefor. The reactants can be on opposing walls of the test well, or one can be on the wall and the other on the membrane. In a preferred embodiment, both are coated on the membrane in the test well, one on top of the other. For example, insolubilized receptor (attached to particles) can be applied to the membrane and dried, after which the antigen is coated.

It has been found useful for improved stability of the target ligand in the test well for it to be mixed with a hydrophilic binder of some type, such as a homo- or copolymer of N-vinyl-2-pyrrolidone. Particularly useful copolymers have at least 50% by weight of units derived from the noted ethylenically unsaturated polymerable monomer. These polymers have been observed to provide more defined coatings of the ligand when applied to coatings of insolubilized receptor already on the membrane. The dried mixture becomes permeable when test specimen or other fluid is added. Preparation of the homo- or copolymers can be achieved using standard solution polymerization methods. Target ligand can be an intact organism or a component derived therefrom. The amount of binder and ligand in the mixture can be widely varied according to practical considerations of cost, sensitivity of the receptor and ease of coating the membrane. Generally, the binder is present at from 1 to 10 weight percent of the solution. The ratio of target ligand to binder is from 10⁴ to 10¹⁰ cells per 0.05 gram of binder.

In one embodiment, the target ligand is an antigen extracted for a microorganism associated with a periodontal disease. Thus, the positive control test well contains a mixture of binder and the antigen of interest.

The test device of this invention can be provided individually or preferably as part of a diagnostic test kit having individually packaged components, reagents, assay equipment and instructions.

The following examples are included to illustrate the practice of this invention. All percentages are by weight unless otherwise noted.

SURECELL™ disposable test devices were used containing LOPRODYNE™ nylon microporous filtration membranes (Pall Corp., various average pore sizes) incorporated into the three test wells. Some membranes (5 µm LOPRODYNE™) were used after coating with FC™ 135 nonionic surfactant (3M Corporation).

The wash solution comprised sodium decyl sulfate (1.8%) in phosphate buffered saline solution buffer (pH 7.2).

A dye-providing composition was prepared to include 4,5-bis(4-methoxyphenyl)-2-(3,5-dimethoxy-4-hydroxyphenyl)imidazole leuco dye (0.008%), poly(vinyl pyrrolidone) (1%), sodium phosphate buffer (10 mmolar, pH 6.8), hydrogen peroxide (10 mmolar), 4'-hydroxyacetanilide (0.5 mmolar) and diethylenetriaminepentaacetic acid (10 µmotar).

Polyclonal antibodies directed against each of the three microorganisms Actinobacillus actinomycetemcomitans (A.a.), Prevotella intermedia (P.i.) and Porphyromonas gingivalis (P.g.) were prepared by intravenous injection of rabbits. IgG fractions were prepared by ammonium sulfate precipitation, and stored at 4°C in phosphate buffered saline solution (0.3-0.4% solution). The bacterial strains used to produce the antisera were supplied as viable cultures by H.S. Reynolds (SUNY, Buffalo School of Dentistry). Isolates were subcultured on anaerobic plates. The microorganisms were those identified by the deposit numbers of ATCC 43717, ATCC 43718 and ATCC 43719 for A.a. (serotypes A, B and C, respectively), ATCC 25611, NCTC 9336 and ATCC 49046 for P.i. (serotypes A, B and C, respectively) and ATCC 33277, ATCC 53978 and ATCC 53977 for P.g. (serotypes A, B and C, respectively). ATCC is the American Type Culture Collection (Rockville, Maryland) and NCTC is the National Collection of Type Cultures (London, U.K.).

Water insoluble reagents were prepared by covalently binding antibodies specific to each microorganism (all of serotypes A, B and C) to polymeric particles (1 µmeter average diameter) of poly[styrene-co-4-(2-chloroethylsulfonylmethyl)styrene] (95.5:4.5 molar ratio) which had been prepared using the procedures of EP-A-0 323 692. Covalent attachment was achieved by adding the antibodies specific to a given microorganism (0.75 mg/ml final solution with 0.25 mg/ml of each serotype A, B and C) to a solution of borate buffer (0.05 molar, pH 8.5) in a test tube and mixing well. The polymeric particles (3% solids, about 1 µl average diameter) were added to the buffered mixture, and the resulting suspension was rotated end-over-end for 4-24 hours at room temperature to allow covalent attachment of the antibodies to the particles. The suspension was then centrifuged at 2800 rpm for 10 minutes. The supernatant was discarded and the pellet was suspended in glycine buffer (0.1%, pH 8.5) containing merthiolate (0.01%).

A coating suspension of the reagent described above (0.95% solids) was prepared to have polyacrylamide binder (5%), in glycine buffer (0.1 molar, pH 8.5). Each reagent directed to a distinct antigen was coated in defined regions of the membrane in the test devices described above.

Enzyme-antibody conjugates were prepared using antibodies directed to each microorganism conjugated to horseradish peroxidase using the procedure of Yoshitake et al, Eur.J.Biochem., 101, 395, 1979. Each conjugate composition comprised the conjugates (7.5-15 µg of each antibody per ml for Examples 1-5, and 5.6-11.3 µg of each antibody per ml for Example 6) added to a solution of casein [0.5%, from a 1% solution in 0.1 molar 3-(N-morpholino)propanesulfonic acid buffer, pH 7.5], TWEEN™ 20 nonionic surfactant (0.3% ICI Americas, Inc.), merthiolate (0.01%), 4'-hydroxyacetanilide (10 mmolar) in buffer (0.1 molar, pH 7.5). The amount of antibody specific for A.a. (all serotypes) and P.g. (all serotypes) was 10 µg/ml. For P.i. (serotypes A and C), the amount was 7.5 µg/ml, and for serotype B of this microorganism, it was 15 µg/ml.

Absorbents used in the fluid collecting compartments of the test devices are identified as follows:
"R12022" absorbent purchased from American Filtrona Company having a density of about 2.2 g/cm³.

"R13528" absrobent purchased from American Filtrona Company having a density of about 1.8 g/cm³.

"R13531" absorbent purchased from American Filtrona Company having a density of about 1.6 g/cm³.

All other reagents were obtained from Eastman Kodak Company or other well known suppliers of chemicals and reagents.

### Assay Protocol:

This protocol was used in all of the examples. A solution (400 µl) of sodium dodecyl sulfate (5%) and EMCOL™ CC-9 cationic surfactant (5%, Witco Chemical Co.) in glycine buffer (0.1 molar, pH 8.5) was added to the test wells of the test devices. A sample (about 40 µl) of the peroxidase-labeled conjugate was added to each test well followed by incubation at room temperature for 2 minutes.

Wash solution (500 µl) was added to each test well (filling them halfway) and allowed to drain. This was repeated once.

After drainage of the last wash solution, the dye-providing composition (80 µl) was added to each test well followed by incubation at room temperature for one minute. The resulting dye signal was then observed and compared to a color intensity score card having values of 1 to 10 with 10 representing the highest dye density. A value was also given to the background observed in the region surrounding the dye signal. The dye signals as reflection densities were also converted to transmission density (D_{T}) values using the conventional Williams-Clapper transformation (see J.OpticalSoc.Am., 43, 595, 1953).

### Examples 1-5: Assays Using Various Combinations of Membranes and Absorbing Means

Assays were carried out using the protocol described above using test devices having various combinations of absorbing means and membranes with differing average pore sizes. The assays are identified as follows:
Control A: Membrane was surfactant coated and had an average pore size of 5 µmeters. The absorbing means was that identified above as "R12022".

Control B: Membrane was surfactant coated and had an average pore size of 5 µmeters. The absorbing means was that identified above as "R13528".

Example 1: Membrane was uncoated and had an average pore size of 3 µmeters. The absorbing means was that identified above as "R13528".

Example 2: Membrane was uncoated and had an average pore size of 1.2 µmeters. The absorbing means was that identified above as "R12022".

Example 3: Membrane was uncoated and had an average pore size of 1.2 µmeters. The absorbing means was that identified above as "R13531".

Example 4: Membrane was uncoated and had an average pore size of 1.2 µmeters. The absorbing means was that identified above as "R13528".

Example 5: Membrane was uncoated and had an average pore size of 0.45 µmeter. The absorbing means was that identified above as "R12022".

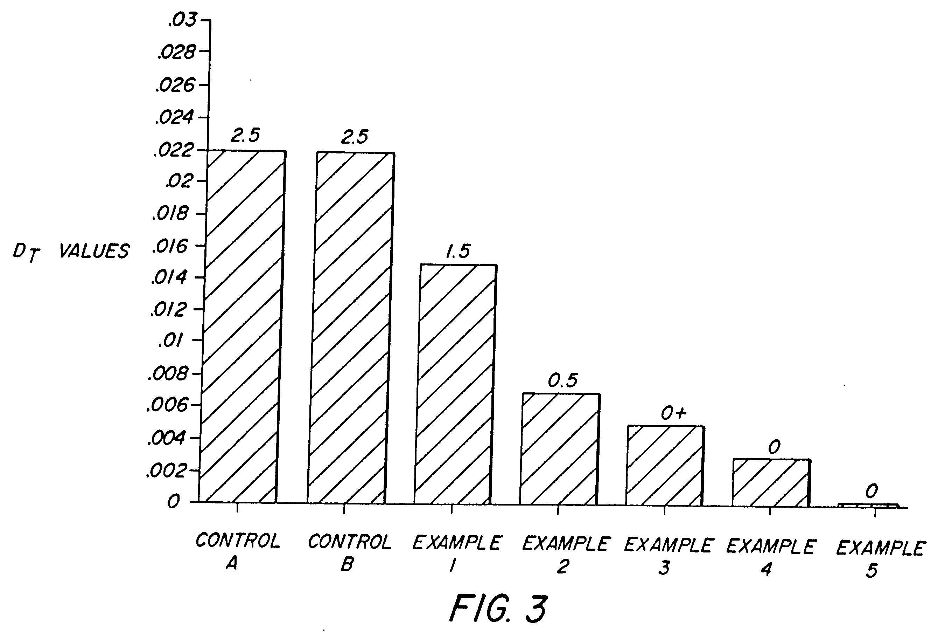

The results of the assays are illustrated in the bar graphs of FIG. 3. The D_{T} values for the background are noted on the side of the figure and the score card values are noted on each bar. It is clear that decreasing the average pore size of the membrane is the most important factor in reducing background signal in the assays.

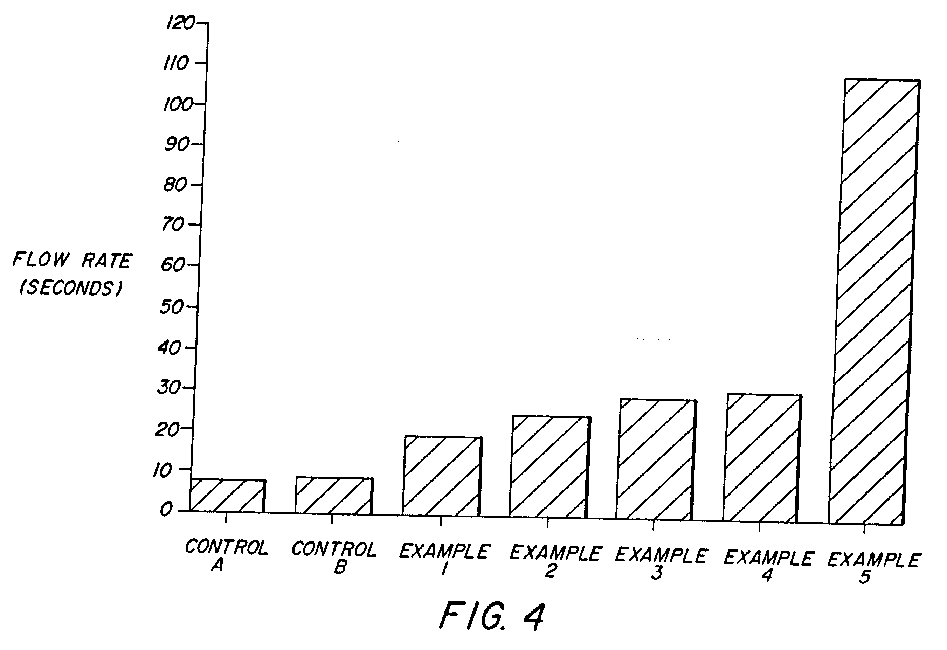

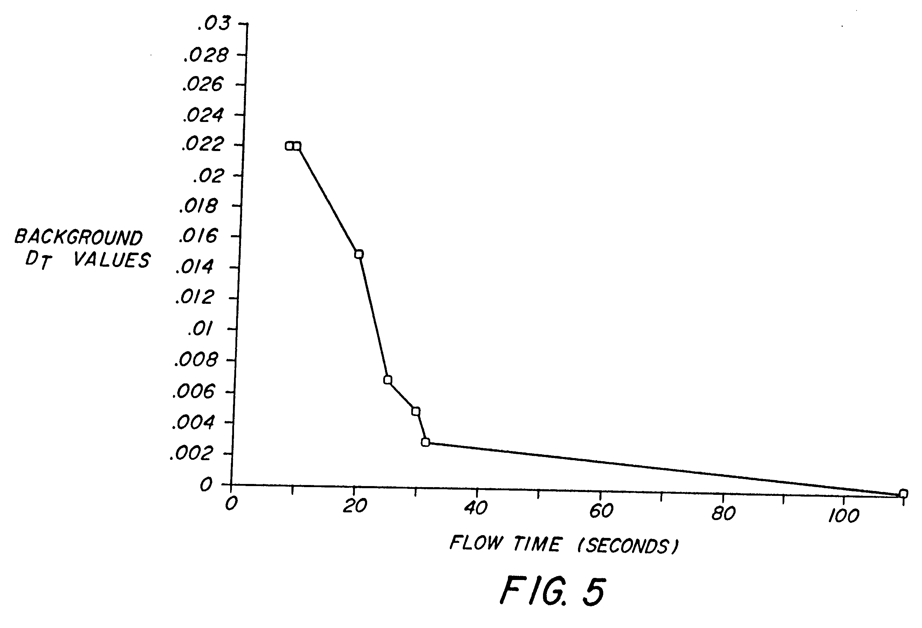

FIG.4 illustrates the time (seconds) for a fluid sample (500 µl) containing sodium dodecyl sulfate (5%) and EMCOL™ CC-9 cationic surfactant (5%) in glycine buffer (0.1 molar, pH 8.5) to completely drain through the test wells of the test devices. The test devices (Examples 1-5) providing acceptably low background had a flow time of at least about 20 seconds. These results are also seen in the graphical plot of FIG. 5.

### Example 6 Preparation of Positive Control in Test Device and Assay Using Same

This example shows the use of various water-soluble binders in admixture with microorganisms in the preparation of positive control reagents in a test device. It was observed that merely applying the microorganism to a coating of antibody-bead reagent on the membranes resulted in positive control dye images that were blurred. Certain binders reduced or eliminated the blurred images.

The following polymers were evaluated as binders:
Polymer A: Polyacrylamide,
Polymer B: Poly[acrylamide-co-N-(3-acetoacetamidopropyl)methacrylamide] (95:5 weight ratio),
Polymer C: Polyvinylpyrrolidone (360,000 molecular weight), and
Polymer D: Polyvinylpyrrolidone (40,000 molecular weight).

A stock solution (100 µl) of each of the three serotypes of A.a., P.g. and P.i. were combined with phosphate buffered saline solution (700 µl). Each stock solution contained 10⁹ cells/ml and merthiolate (0.2%) in phosphate buffered saline solution. The resulting antigen solutions were applied to the membrane using a pipette.

Separate antigen solutions were prepared for deposition by printing on the membranes. A separate antigen solution of all three of the A.a. serotypes (100 µl of each serotype stock solution) was combined with phosphate buffered saline solution (700 µl).

Each of the antigen solutions noted above was mixed with a polymer binder solution containing fluorescein (to facilitate detection of the deposited mixtures). The resulting mixtures contained one part by volume of antigen solution to 2.5 parts by volume of binder solution [containing fluorescein (0.05%) and polymer in the amounts indicated in the tables below].

This example was carried out using SURECELL™ disposable test devices containing LOPRODYNE™ filtration membranes (1.2 µm pore size) in the test wells and "R13531" absorbent underneath the membranes. Some membranes were tested in uncoated form while others were tested after being coated with a suspension of reagents composed of particles having antibody attached thereto. The reagent was deposited as a suspension (24 µl) thereon using a pipette and allowed to dry. The antibody-bead reagents were prepared as follows:
Water-insoluble reagents were prepared by covalently binding antibodies (prepared as described above) specific to all serotypes of A.a., P.i. and P.g. to particles (1 µm average diameter) of poly[styrene-co-4-(2-chloroethylsulfonylmethyl)styrene] (95.5:4.5 molar ratio) prepared using the procedures described in EP-A-0 323 692. Covalent attachment was achieved by adding the antibodies specific to a given microorganism (0.75 mg/ml final solution with 0.25 mg/ml of each serotype A, B and C of P.g., 0.52 mg/ml final solution with 0.17 mg/ml of each serotype A, B, and C of A.a., and 0.52 mg/ml final solution with 0.13 mg/ml of each serotype A and C of P.i. and 0.26 mg/ml of serotype B of P.i.) to a solution of borate buffer (0.05 molar, pH 8.5) in a test tube and mixing well. The particles (3% solids) were added to the buffered mixture, and the resulting suspension was rotated end-over-end for 4-24 hours at room temperature to allow covalent attachment. The resulting suspension of reagent was centrifuged at 2800 rpm for 10 minutes. The supernatant was then discarded and the pellet resuspended in glycine buffer (0.1%, pH 8.5) containing merthiolate (0.01%).

A coating suspension of the water-insoluble reagent described above (0.95% solids) was prepared containing polyacrylamide binder (5%) in glycine buffer (0.1 molar, pH 8.5). A sample (23 mg) of this suspension was coated on some of the membranes and dried.

In screening the positive control reagents, the reagent (1.5-1.8 µl) was deposited onto the membranes (coated or uncoated) by forming a pendant drop on the tip of a pipette and contacting the pendant drop to the membrane surface, thus allowing the capillarity of the membrane to draw the fluid of the reagent off the pipette tip and to absorb the fluid. After the positive control reagent was dried, each was tested for image formation by performing the following assay procedure:
A modified sample (56 µl) of the peroxidase-labeled conjugate described above was added to each test well followed by incubation for two minutes at room temperature. The conjugate solution was modified by having 25% less antibody, the buffer was phosphate buffered saline solution (0.05 molar, pH 7.3) and contained additionally LONZAINE™ C amphoteric surfactant (0.0035%, Lonza Corp.).

Wash solution (about 500 µl) was added and allowed to drain twice. This solution contained TERGITOL™ 4 anionic surfactant (1.35%, Union Carbide Corp.), casein (0.5%) and merthiolate (0.01%) in glycine buffer (0.1 molar, pH 10).

After drainage of the last wash, the dye-providing composition (120 µl) was added to each test well followed by incubation at room temperature for one minute. The dye-providing composition was that described above, except that 4'-hydroxyacetanilide was present at 5 mmolar.

The resulting dye signal was then observed and compared to a color intensity score card having values of 1 to 10 with 10 representing the highest dye density. The results are recorded in Table I below.

The developed dye signal can vary in size and shape. For example, it can form a clear concentrated single dot which is preferred. Alternatively, the dye may migrate to form a broad concentric band of undesired density around a center dot. The center dot and band are contrasted with each other as well as with an outer ring of higher unwanted dye density. The contrast between the center spot and the broad band is most important. Thus, the results in Table I are presented as dye scores in the center spot, the broad band and the outer ring. The best results are obtained when the difference in color scores between the center spot and the broad band is maximum. The results indicate that polyacrylamide and polyvinylpyrrolidone provided the best results.

**TABLE I**

| POLYMER BINDER (CONCENTRATION WEIGHT %) | DYE SIGNALS | | | | | |
|---|---|---|---|---|---|---|
| | UNCOATED MEMBRANE | | | REAGENT COATED MEMBRANE | | |
| | CENTER SPOT | INNER BAND | OUTER RING | CENTER SPOT | INNER BAND | OUTER RING |
| A (13.5%) | 4 | 1 | 4 | 7 | 4 | 8 |
| B (10 %) | 5 | 3 | 5 | 8.5 | 6-6.5 | 8 |
| C (7%) | 4 | 0.5 | 3.5 | 8 | 6 | 7.5 |
| D (10%) | 4 | 0 | 0 | 8 | 6.5 | 7 |

A preferred process for applying the positive control reagent is by printing. The reagent is pumped through a porous applicator having a printing surface configured into a specific pattern. As the printing surface is contacted with the reagent-coated membrane, the positive control reagent is transferred thereto in a specific printed pattern consistent with the shape of the applicator.

Dye signal images were developed by the procedure described above. A rating of the printed dye image was made from 1 to 5 with 5 representing the best developed signal pattern. The dye signal images can vary in intensity and dimensions of transferred pattern. It is preferred that the dimensions of the pattern match the dimensions of the porous printing surface.

Only Polymers A, C and D were tested as binders using this procedure. The results are provided in Table II below. It can be seen that Polymer C is preferred although Polymer D provided acceptable results as well.

**TABLE II**

| POLYMER BINDER (CONCENTRATION WEIGHT %) | DYE SIGNAL SCORE |
|---|---|
| A | 1 |
| C | 5 |
| D | 3 |

## Claims

1. A method for the determination of a specific binding ligand comprising the steps of:
A. forming an insolubilized complex of a target specific binding ligand and a receptor specific therefor on one surface of a microporous filtration membrane which is in fluid contact on the opposing surface thereof with a means for absorbing fluid passing through the membrane,
B. removing uncomplexed materials from the insolubilized complex by washing them through the membrane into the absorbing means with a wash solution comprising a wash reagent,
provided that the average pore size of the membrane and the density or hydrophilicity of the absorbing means are such that, the time for washing is at least 20 seconds, and
C. detecting the insolubilized complex on the membrane as an indication of the determination of the target specific binding ligand.

2. The method as claimed in claim 1 wherein, for a given volume of fluid, the time for washing is from 25 to 45 seconds.

3. The method as claimed in any of claims 1 and 2 wherein the ligand is also reacted with a second receptor therefor which is water-soluble and detectably labeled.

4. The method as claimed in any of claims 1 to 3 for the determination of any of Actinobacillus actinomycetemcomitans, Prevotella intermedia and Porphyromonas gingivalis.

5. The method as claimed in any of claims 1 to 4 for the simultaneous determination of two or more specific binding ligands in distinct regions on the membrane surface by complexing the ligands with receptors specific for the ligands to form distinct insolubilized complexes in the distinct regions.

6. A test device comprising a test well comprising:
(a) an upper reaction compartment (12),
(b) a lower fluid collection compartment (14), and
(c) a microporous filtration membrane (16) separating the compartments,
the lower fluid collection compartment (14) containing means for absorbing fluid (30) from the upper compartment (12) to the lower compartment (14) through the membrane (16), the absorbing means (30) being in fluid contact with the membrane (16),
the test device characterized wherein the membrane (16) has an average pore size and the absorbing means (30) has a density or hydrophilicity such that, the time for a given volume of fluid to pass through the membrane (16) is at least 20 seconds.

7. A diagnostic test kit comprising:
(1) receptor molecules for a target specific binding ligand, and
(2) a wash solution comprising a wash reagent,
the test kit characterized wherein it further comprises a test device as claimed in claim 6.

8. The kit as claimed in claim 7 wherein the receptor molecules are antibodies specific to any of Actinobacillus actinomycetemcomitans, Prevotella intermedia and Porphyromonas gingivalis.

9. The invention as claimed in any of claims 6 to 8 wherein the test device comprises at least two test wells, each having components (a), (b) and (c) as defined,
provided one of the test wells comprises a dried mixture of a specific binding ligand and a polymeric binder material.

10. The invention as claimed in claim 9 wherein the dried mixture comprises any of Actinobacillus actinomycetemcomitans, Prevotella intermedia and Porphyromonas gingivalis or an antigen extracted therefrom.

11. The invention as claimed in any of claims 9 and 10 wherein the binder material is a polymer having at least 50% by weight of units derived from N-vinyl-2-pyrrolidone.

12. The invention as claimed in any of claims 1 to 11 wherein the average pore size of the membrane is from 0.1 to 3 µm.

13. The invention as claimed in any of claims 1 to 12 wherein the absorbing means has a density of from 1.2 to 2.5 g/cm³.

14. The invention as claimed in any of claims 1 to 13 wherein the absorbing means is in physical contact with the membrane.
